# EUROPEAN PATENT APPLICATION

(11) **EP 1 658 868 A1**
(43) Date of publication of application: **24.05.2006**
(21) Application number: 05257061.1
(22) Date of filing: 16.11.2005
(51) Int. Cl.: A61M 1/00, A61B 17/34, A61M 16/00

(54) **Devices for controlling collateral ventilation**

(30) Priority: 19.11.2004 US 993748
(71) Applicant: Nitinol Development Corporation, Fremont, California 94539 (US)
(72) Inventor: Tanaka, Don, Saratoga, CA 95070 (US)
(74) Representative: Belcher, Simon James

(57) **Abstract**

Chemical lung volume reduction maybe utilized to control collateral ventilation so that trapped air in diseased lungs can be removed. The chemical or therapeutic agent may be locally delivered to the site or sites of highest collateral ventilation utilizing any number of methods including bronchoscopic delivery. A system for controlling collateral ventilation is described. The system comprises: a device for locally or regionally delivering a drug, agent and/or chemical for constricting channels between alveoli in a diseased area of the lung; and a device for venting air trapped in the lungs for decompressing the diseased area of the lung.

## Description

The present invention relates to systems and methods for treating diseased lungs, and more particularly, to methods and systems for controlling collateral ventilation and removing trapped air in diseased lungs.

As a result of studies that date back to the 1930's and particularly studies conducted in the 1960's and early 1970's, it has been determined that long-term continuous oxygen therapy is beneficial in the treatment of hypoxemic patients with chronic obstructive pulmonary disease. In other words, a patient's life and quality of life can be improved by providing a constant supplemental supply of oxygen to the patient's lungs.

However, with the desire to contain medical costs, there is a growing concern that the additional cost of providing continuous oxygen therapy for chronic lung disease will create an excessive increase in the annual cost of oxygen therapy. Thus, it is desirable that oxygen therapy, when provided, be as cost effective as possible.

The standard treatment for patients requiring supplemental oxygen is still to deliver oxygen from an oxygen source by means of a nasal cannula. Such treatment, however, requires a large amount of oxygen, which is wasteful and can cause soreness and irritation to the nose, as well as being potentially aggravating. Other undesirable effects have also been reported. Various other medical approaches, which have been proposed to help reduce the cost of continuous oxygen therapy, have been studied.

Various devices and methods have been devised for performing emergency cricothyroidotomies and for providing a tracheotomy tube so that a patient whose airway is otherwise blocked may continue to breath. Such devices are generally intended only for use with a patient who is not breathing spontaneously and are not suitable for the long term treatment of chronic lung disease. Typically, such devices are installed by puncturing the skin to create a hole into the cricoid membrane of the larynx above the trachea into which a relatively large curved tracheotomy tube is inserted. As previously described, the use of such tubes has been restricted medically to emergency situations where the patient would otherwise suffocate due to the blockage of the airway. Such emergency tracheotomy tubes are not suitable for long term therapy after the airway blockage is removed.

Other devices which have been found satisfactory for emergency or ventilator use are described in US-953922 to Rogers; US-2873742 to Shelden; US-3384087 to Brummelkamp; US-3511243 to Toy; US-3556103 to Calhoun; US-2991787 to Shelden, et al; US-3688773 to Weiss; US-3817250 to Weiss, et al.; and US-3916903 to Pozzi.

Although tracheotomy tubes are satisfactory for their intended purpose, they are not intended for chronic usage by outpatients as a means for delivering supplemental oxygen to spontaneously breathing patients with chronic obstructive pulmonary disease. Such tracheotomy tubes are generally designed so as to provide the total air supply to the patient for a relatively short period of time. The tracheotomy tubes are generally of rigid or semi-rigid construction and of caliber ranging from 2.5 mm outside diameter in infants to 15 mm outside diameter in adults. They are normally inserted in an operating room as a surgical procedure or during emergency situations, through the crico-thyroid membrane where the tissue is less vascular and the possibility of bleeding is reduced. These devices are intended to permit passage of air in both directions until normal breathing has been restored by other means.

Another type of tracheotomy tube is disclosed in Jacobs, US-3682166 and US-3788326. The catheter described therein is placed over 14 or 16-gauge needle and inserted through the crico-thyroid membrane for supplying air or oxygen and vacuum on an emergency basis to restore the breathing of a non-breathing patient. The air or oxygen is supplied at 207 to 689 kPa (30 to 100 psi) for inflation and deflation of the patient's lungs. The Jacobs catheter, like the other tracheotomy tubes previously used, is not suitable for long-term outpatient use, and could not easily be adapted to such use.

Due to the limited functionality of tracheotomy tubes, transtracheal catheters have been proposed and used for long term supplemental oxygen therapy. For example the small diameter transtracheal catheter (16 gauge) developed by Dr. Henry J. Heimlich (described in THE ANNALS OF OTOLOGY, RHINOLOGY & LARYNGOLOGY, November-December 1982; Respiratory Rehabilitation with Transtracheal Oxygen System) has been used by the insertion of a relatively large cutting needle (14 gauge) into the trachea at the mid-point between the cricothyroid membrane and the sternal notch. This catheter size can supply oxygen up to about 3 liters per minute at low pressures, such as 13.8 kPa (2 psi) which may be insufficient for patients who require higher flow rates. It does not, however, lend itself to outpatient use and maintenance, such as periodic removal and cleaning, primarily because the connector between the catheter and the oxygen supply hose is adjacent and against the anterior portion of the trachea and cannot be easily seen and manipulated by the patient. Furthermore, the catheter is not provided with positive means to protect against kinking or collapsing which would prevent its effective use on an outpatient basis. Such a feature is not only desirable but necessary for long term outpatient and home care use. Also, because of its structure, i.e. only one exit opening, the oxygen from the catheter is directed straight down the trachea toward the bifurcation between the bronchi. Because of the normal anatomy of the bronchi wherein the left bronchus is at a more acute angle to the trachea than the right bronchus, more of the oxygen from that catheter tends to be directed into the right bronchus rather than being directed or mixed for more equal utilization by both bronchi. Also, as structured, the oxygen can strike the carina, resulting in an undesirable tickling sensation and cough. In addition, in such devices, if a substantial portion of the oxygen is directed against the back wall of the trachea it may cause erosion of the mucosa in this area which in turn may cause chapping and bleeding. Overall, because of the limited output from the device, it may not operate to supply sufficient supplemental oxygen when the patient is exercising or otherwise quite active or has severe disease.

Diseases associated with chronic obstructive pulmonary disease include chronic bronchitis and emphysema. One aspect of an emphysematous lung is that the communicating flow of air between neighboring air sacs is much more prevalent as compared to healthy lungs. This phenomenon is known as collateral ventilation. Another aspect of an emphysematous lung is that air cannot be expelled from the native airways due to the loss of tissue elastic recoil and radial support of the airways. Essentially, the loss of elastic recoil of the lung tissue contributes to the inability of individuals to exhale completely. The loss of radial support of the airways also allows a collapsing phenomenon to occur during the expiratory phase of breathing. This collapsing phenomenon also intensifies the inability for individuals to exhale completely. As the inability to exhale completely increases, residual volume in the lungs also increases. This then causes the lung to establish in a hyperinflated state where an individual can only take short shallow breaths. Essentially, air is not effectively expelled and stale air accumulates in the lungs. Once the stale air accumulates in the lungs, the individual is deprived of oxygen.

Currently, treatments for chronic obstructive pulmonary disease include bronchodilating drugs, oxygen therapy as described above, and lung volume reduction surgery. Bronchodilating drugs only work on a percentage of patients with chronic obstructive pulmonary disease and generally only provides short-term relief. Oxygen therapy is impractical for the reasons described above, and lung volume reduction surgery is an extremely traumatic procedure that involves removing part of the lung. The long term benefits of lung volume reduction surgery are not fully known.

Accordingly, there exists a need for substantially reducing collateral ventilation by reducing the flow of air between neighboring air sacs so that a less invasive technique of lung volume reduction surgery may be accomplished. This technique involves venting trapped gases from the lung or lungs to reduce the volume of a specific location of the lung without having to resect any tissue. Collateral ventilation will only counter the removal of localized air in the lung by allowing air from other parts of the lung to replace the removed air.

The present invention overcomes the limitations in treating diseases associated with chronic obstructive pulmonary disorders as briefly described above.

In accordance with one aspect, the present invention provides a system for controlling collateral ventilation comprising:
a device for locally or regionally delivering a drug, agent and/or chemical for constricting channels between alveoli in a diseased area of the lung; and
a device for venting air trapped in the lungs for decompressing the diseased area of the lung.

The present invention is can be used in a method for controlling collateral ventilation. The method comprises the local/regional delivery, via native airways, of an agent for temporarily constricting channels between alveoli in a diseased area of the lung and venting trapped gases in the diseased area of the lung via the same or different native airways to decompress a specific location of a diseased area of the lung.

The present invention utilizes therapeutic agents to temporarily constrict collateral ventilation pathways, thereby achieving in essence, the ability to conduct lung volume reduction without the resection of lung tissue. The therapeutic agent or agents may be delivered via any number of ways to the appropriate site or sites in the lung. This local delivery enhances the effectiveness of the procedure while reducing the risks associated with systemic drug delivery.

Air typically enters the mammalian body through the nostrils and flows into the nasal cavities. As the air passes through the nostrils and nasal cavities, it is filtered, moistened and raised or lowered to approximately body temperature. The back of the nasal cavities is continuous with the pharynx (throat region); therefore, air may reach the pharynx from the nasal cavities or from the mouth. Accordingly, if equipped, the mammal may breath through its nose or mouth. Generally air from the mouth is not as filtered or temperature regulated as air from the nostrils. The air in the pharynx flows from an opening in the floor of the pharynx and into the larynx (voice box). The epiglottis automatically closes off the larynx during swallowing so that solids and/or liquids enter the esophagus rather than the lower air passageways or airways. From the larynx, the air passes into the trachea, which divides into two branches, referred to as the bronchi. The bronchi are connected to the lungs.

The lungs are large, paired, spongy, elastic organs, which are positioned in the thoracic cavity. The lungs are in contact with the walls of the thoracic cavity. In humans, the right lung comprises three lobes and the left lung comprises two lobes. Lungs are paired in all mammals, but the number of lobes or sections of lungs varies from mammal to mammal. Healthy lungs, as discussed below, have a tremendous surface area for gas/air exchange. Both the left and right lung is covered with a pleural membrane. Essentially, the pleural membrane around each lung forms a continuous sac that encloses the lung. A pleural membrane also forms a lining for the thoracic cavity. The space between the pleural membrane forming the lining of the thoracic cavity and the pleural membranes enclosing the lungs is referred to as the pleural cavity. The pleural cavity comprises a film of fluid that serves as a lubricant between the lungs and the chest wall.

In the lungs, the bronchi branch into a multiplicity of smaller vessels referred to as bronchioles. Typically, there are more than one million bronchioles in each lung. Each bronchiole ends in a cluster of extremely small air sacs referred to as alveoli. An extremely thin, single layer of epithelial cells lining each alveolus wall and an extremely thin, single layer of epithelial cells lining the capillary walls separate the air/gas in the alveolus from the blood. Oxygen molecules in higher concentration pass by simple diffusion through the two thin layers from the alveoli into the blood in the pulmonary capillaries. Simultaneously, carbon dioxide molecules in higher concentration pass by simple diffusion through the two thin layers from the blood in the pulmonary capillaries into the alveoli.

Breathing is a mechanical process involving inspiration and expiration. The thoracic cavity is normally a closed system and air cannot enter or leave the lungs except through the trachea. If the chest wall is somehow compromised and air/gas enters the pleural cavity, the lungs will typically collapse. When the volume of the thoracic cavity is increased by the contraction of the diaphragm, the volume of the lungs is also increased. As the volume of the lungs increase, the pressure of the air in the lungs falls slightly below the pressure of the air external to the body (ambient air pressure). Accordingly, as a result of this slight pressure differential, external or ambient air flows through the respiratory passageways described above and fills the lungs until the pressure equalizes. This process is inspiration. When the diaphragm is relaxed, the volume of the thoracic cavity decreases, which in turn decreases the volume of the lungs. As the volume of the lungs decrease, the pressure of the air in the lungs rises slightly above the pressure of the air external to the body. Accordingly, as a result of this slight pressure differential, the air in the alveoli is expelled through the respiratory passageways until the pressure equalizes. This process is expiration.

Continued insult to the respiratory system may result in various diseases, for example, chronic obstructive pulmonary disease. Chronic obstructive pulmonary disease is a persistent obstruction of the airways caused by chronic bronchitis and pulmonary emphysema. In the United States alone, approximately fourteen million people suffer from some form of chronic obstructive pulmonary disease and it is in the top ten leading causes of death.

Chronic bronchitis and acute bronchitis share certain similar characteristics; however, they are distinct diseases. Both chronic and acute bronchitis involve inflammation and constriction of the bronchial tubes and the bronchioles; however, acute bronchitis is generally associated with a viral and/or bacterial infection and its duration is typically much shorter than chronic bronchitis. In chronic bronchitis, the bronchial tubes secrete too much mucus as part of the body's defensive mechanisms to inhaled foreign substances. Mucus membranes comprising ciliated cells (hair like structures) line the trachea and bronchi. The ciliated cells or cilia continuously push or sweep the mucus secreted from the mucus membranes in a direction away from the lungs and into the pharynx, where it is periodically swallowed. This sweeping action of the cilia, functions to keep foreign matter from reaching the lungs. Foreign matter that is not filtered by the nose and larynx, as described above, becomes trapped in the mucus and is propelled by the cilia into the pharynx. When too much mucus is secreted, the ciliated cells may become damaged, leading to a decrease in the efficiency of the cilia to sweep the bronchial tubes and trachea of the mucus containing the foreign matter. This in turn causes the bronchioles to become constricted and inflamed and the individual becomes short of breath. In addition, the individual will develop a chronic cough as a means of attempting to clear the airways of excess mucus.

Individuals who suffer from chronic bronchitis may develop pulmonary emphysema. Pulmonary emphysema is a disease in which the alveoli walls, which are normally fairly rigid structures, are destroyed. The destruction of the alveoli walls is irreversible. Pulmonary emphysema may be caused by a number of factors, including chronic bronchitis, long term exposure to inhaled irritants, e.g. air pollution, which damage the cilia, enzyme deficiencies and other pathological conditions. In pulmonary emphysema, the alveoli of the lungs lose their elasticity, and eventually the walls between adjacent alveoli are destroyed. Accordingly, as more and more alveoli walls are lost, the air exchange (oxygen and carbon dioxide) surface area of the lungs is reduced until air exchange becomes seriously impaired. The combination of mucus hypersecretion and dynamic airway compression are mechanisms of airflow limitation in chronic obstructive pulmonary disease. Dynamic airway compression results from the loss of tethering forces exerted on the airway due to the reduction in lung tissue elasticity. Mucus hypersecretion is described above with respect to bronchitis. In other words, the breakdown of lung tissue leads to the reduced ability of the lungs to recoil and the loss of radial support of the airways. Consequently, the loss of elastic recoil of the lung tissue contributes to the inability of individuals to exhale completely. The loss of radial support of the airways also allows a collapsing phenomenon to occur during the expiratory phase of breathing. This collapsing phenomenon also intensifies the inability for individuals to exhale completely. As the inability to exhale completely increases, residual volume in the lungs also increases. This then causes the lung to establish in a hyperinflated state where an individual can only take short shallow breaths. Essentially, air is not effectively expelled and stale air accumulates in the lungs. Once the stale air accumulates in the lungs, the individual is deprived of oxygen. There is no cure for pulmonary emphysema, only various treatments, including exercise, drug therapy, such as bronchodilating agents, lung volume reduction surgery and long term oxygen therapy.

As stated above, one means for controlling collateral ventilation involved lung volume reduction surgery. Generally speaking, lung reduction surgery is an extremely traumatic procedure that involves removing part or parts of the lung or lungs. By removing the portion of the lung or lungs which is hyperinflated, pulmonary function may improve due to a number of mechanisms, including enhanced elastic recoil, correction of ventilation/perfusion animation and improved efficacy of respiratory work. Essentially, as diseased tissue volume is reduced, the healthier tissue is better ventilated. However, lung volume reduction surgery possesses a number of potential risks. Less drastic and less invasive techniques that may be utilized include bronchoscopic lung volume reduction surgery. This would allow compression of the most diseased part of the lung without the trauma of a surgical resection. Essentially, this technique involves obstructing inward flow of air through specific native airways to "suck down" local areas of the lung. An airway implant is the device used to prevent the inward flow of air into the lung.

Collateral ventilation, which as described above, is the flow of air between segments of the lung, makes it difficult to locally "suck down" a specific part of the lung. In addition, emphysema patients have a greater degree of collateral ventilation than a normal or healthy individual. This higher degree of collateral ventilation makes it more difficult to locally "suck down" part of the lung.

In accordance with one aspect of the present invention, in order to control the amount of collateral ventilation in the lungs, drugs, agents and/or chemicals may be utilized to constrict the collateral pathways. These drugs, agents and/or chemicals, which may be locally delivered through a bronchoscope, may temporarily constrict the collateral pathways, and effectively allow the "suck down" process. The devices and methods to accomplish the "suck down" has been published in prior art. These include embodiments where one-way valves are implanted in the airways or adhesives/surfactants are used to "suck down" the lung. Once the localized area is sucked down, a process called atelectasis occurs. Once this occurs, the lung will remain in the compressed state, and bronchoscopic lung volume reduction is achieved. Generally speaking, atelectasis is the collapse of part or all of a lung.

Essentially, in the present invention, the areas of greatest collateral ventilation are determined and then a drug, agent and/or chemical is locally delivered to constrict the collateral channels thereby allowing the portion or portions of the lung to collapse, that is/are ineffective. In other words, a chemical lung volume reduction is achieved even with the counter-acting phenomenom of collateral ventilation.

Various methods may be utilized to determine the location or locations of the diseased tissue, for example, computerized axial tomography or CAT scans, magnetic response imaging or MRI, position emission tomograph or PET, and/or standard X-ray imaging. Once the area or areas of diseased tissue are located, the drugs, agents and/or chemicals may be delivered. It is important to note that the drug, agent and/or chemical may be delivered in any number of ways. For example, while bronchoscopic delivery is safe and effective, the lungs may be accessed via chest tubes or similar devices through the thoracic wall.

Embodiments of the invention will now be described by way of example with reference to the accompanying drawings, in which:
Figure 1 is a diagrammatic representation of the lung and associated airways in accordance with the present invention.
Figure 2 is a diagrammatic representation of the alveolar sacs of a diseased lung with untreated alveolar collateral ventilation in accordance with the present invention.
Figure 3 is a diagrammatic representation of the alveolar sacs of a diseased lung with treated alveolar collateral ventilation in accordance with the present invention.

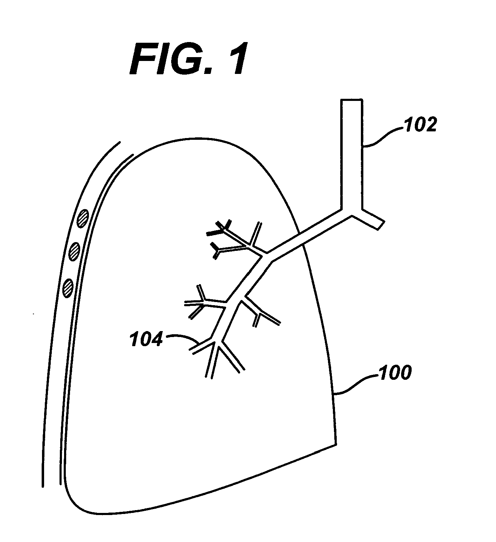

Referring to Figure 1, there is illustrated a lung 100, the trachea 102 and bronchioles 104. The bronchoscope comprising the particular drug, agent and/or chemical, as well as combinations thereof, may be introduced through the trachea, into the one or the bronchi and then as close to the diseased areas in the bronchioles as possible. Once positioned, the drug, agent and/or chemical may be delivered. It is important to note that the drug, agent and/or chemical may be delivered in any number of ways. For example, while brochoscopic delivery is safe and effective, the lungs may be accessed via chest tubes or similar devices.

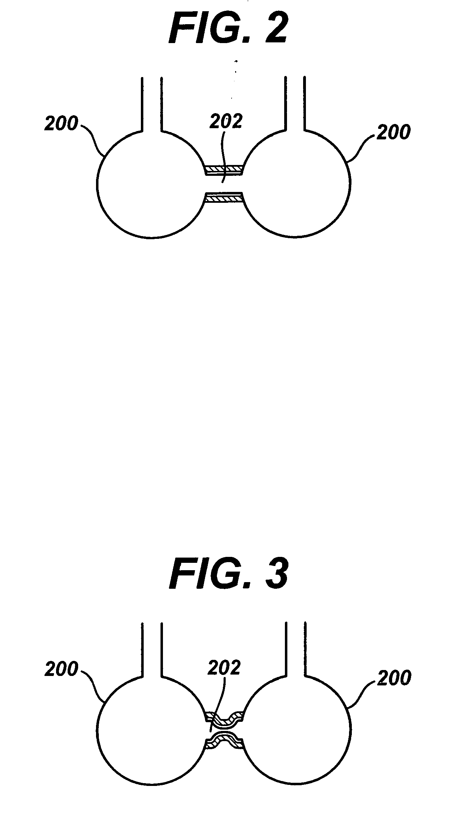

Figure 2 is an enlarged view of the alveolar sacs 200 with a collateral channel 202 extending therebetween. The collateral channel 202 comprises smooth muscle cells. Figure 3 illustrates the same two alveolar sacs 200 with the collateral channel 202 collapsed via smooth muscle cell contraction. Any number of drugs, agents and/or compounds may be utilized to cause smooth muscle cell contraction and are well known in the art. Preferably, any agent utilized will have minimal system impact that is further reduced via local delivery. In the exemplary embodiment, a histamide such as methacholine may be utilized.

While the above described process is utilized to control the amount of collateral ventilation in the lungs via the introduction of chemicals as agents that act to temporarily constrict collateral pathways, the air trapped in the lungs should preferably be vented to decompress the diseased area of the lung. A number of devices and techniques may be utilized to vent the trapped air, including bronchoscopic lung volume reduction surgery utilizing airway one-way valves, bronshoscopic lung volume reduction surgery utilizing sealants and transthoracic lung volume reduction surgery.

Bronchoscopic lung volume reduction surgery may be achieved by utilizing a device delivered utilizing a bronchoscope that will act as a one-way valve, blocking air from entering the diseased portion of the lung. The device will preferably cause the compression and fibrosis of the localized tissue. Bronchoscopic lung volume reduction surgery may be achieved through the use of sealants. In this process, a bronchoscope may be utilized to wash, suction and then seal the diseased portion of the lungs. The process preferably causes the compression and fibrosis of the localized tissue. Transthoracic lung volume reduction surgery is a procedure wherein an alternative exhalation path is made through the thoracic wall using a one-way valve. Once again, this action should preferably cause the compression and fibrosis of the localized tissue.

In accordance with another exemplary embodiment, a conduit or conduits may be positioned in a passage or passages that access the outer pleural layer of the diseased lung or lungs. The conduit or conduits utilize the collateral ventilation of the lung or lungs and allow the trapped air to bypass the native airways and be expelled to the ambient environment or to a containment system outside of the body.

Although shown and described is what is believed to be the most practical and preferred embodiments, it is apparent that departures from specific designs and methods described and shown will suggest themselves to those skilled in the art and may be used without departing from the scope of the invention. The present invention is not restricted to the particular constructions described and illustrated, but should be constructed to cohere with all modifications that may fall within the scope of the appended claims.

## Claims

1. A system for controlling collateral ventilation comprising:
a device for locally or regionally delivering a drug, agent and/or chemical for constricting channels between alveoli in a diseased area of the lung; and
a device for venting air trapped in the lungs for decompressing the diseased area of the lung.

2. A system according to claim 1, wherein the device for delivering a drug, agent and/or chemical is a bronchoscope.

3. A system according to claim 1 or 2, wherein the drug, agent and/or chemical is a histamide.

4. A system according to any one of the preceding claims, wherein the device for venting air trapped in the lungs is a one-way valve for blocking air entering a diseased part of the lungs
